(19) Europäisches Patentamt
European Patent Office
Office européen des brevets

(11) **EP 4 109 467 A1**

(12) **EUROPEAN PATENT APPLICATION**

(43) Date of publication:
**28.12.2022 Bulletin 2022/52**

(21) Application number: **21180580.9**

(22) Date of filing: **21.06.2021**

(51) International Patent Classification (IPC):
**G16H 50/20** (2018.01)    **G16H 40/67** (2018.01)

(52) Cooperative Patent Classification (CPC):
**G16H 50/20; G16H 40/67**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventor: **BONOMI, Alberto Giovanni
5656 AE Eindhoven (NL)**

(74) Representative: **Philips Intellectual Property & Standards
High Tech Campus 52
5656 AG Eindhoven (NL)**

(54) **PATIENT CONTEXT DETECTION**

(57)    There is provided a method and a corresponding system for patient context detection. The method comprises receiving non-geospatial sensor data from at least one sensor of a wearable monitoring device worn by a patient; detecting patient context by processing the non-geospatial sensor data to classify it as belonging to one of at least two predefined patient contexts; and outputting an indication of the detected patient context. Also provided is a method and a corresponding system for training a machine learning model. The method comprises training the machine learning model to classify optionally preprocessed, non-geospatial sensor data received from at least one sensor of a wearable monitoring device worn by a patient as belonging to one of at least two predefined patient contexts.

Fig. 1

**Description**

FIELD OF THE INVENTION

The invention relates to patient context detection and in particular to detecting a semantic location of a patient.

BACKGROUND

Wearable remote monitoring systems monitor a patient's vital signs continuously while sharing data wirelessly with a remote caregiver. Such systems, which are unobtrusive and require minimal to no interaction with the patient, are becoming increasingly popular to improve patient care, reduce hospital costs and boost patient outcome. Continuous monitoring of vital signs such as heart rate and respiration rate can be used to detect clinically relevant health deterioration by means of early warning scoring (EWS) systems. EWS systems use algorithms which apply thresholds to the monitored vital signs to create an early warning score, on the basis of which an alert may be delivered to the caregiver. However, the alerting requirements including the thresholds which are applied when the patient is in hospital may be different from those applied when the patient is at home after discharge. Known patient monitoring systems detect the patient's location using GPS systems or other geolocation methods. However, power consumption and privacy limitations reduce the applicability of these solutions in wearable remote monitoring systems.

SUMMARY

There is therefore a need for more cost effective and privacy-respecting systems and methods for determining patient context, in particular whether the patient being monitored is in hospital or at home, so that remote monitoring services can be adjusted according to the context. This need is met by the subject-matter of the independent claims. Optional features are set forth by the dependent claims.

According to a first aspect, there is provided a method for patient context detection comprising:

receiving non-geospatial sensor data from at least one sensor of a wearable monitoring device worn by a patient;
deriving patient activity level data or posture data from the non-geospatial sensor data;
extracting one or more activity behavior features from the patient activity level data, or extracting one or more posture behavior features from the posture data;
classifying the activity behavior features or posture behavior features as belonging to one of at least two predefined patient contexts; and
outputting an indication of the detected patient context.

The invention is thus able to detect patient context without asking the patient to provide input on their location and without using geolocation services, thereby preserving patient privacy and reducing power consumption.

The term "context" as used herein may be generally understood in the sense of context awareness but refers in particular to patient location, that being a semantic location such as "in hospital" or "at home" rather than a physical or geographic location. "Hospital" is not to be understood in a limiting sense but rather to include any medical facility providing inpatient care.

By "non-geospatial sensor data" is meant sensor data obtained from the at least one sensor of the wearable monitoring device other than that obtained from any GPS sensor or other geolocation sensor that may be present in the wearable. No connection to any remote device is used to determine the location of the subject. In one example, the non-geospatial sensor data comprises accelerometer data or motion data obtained by one or more accelerometers or other motion sensors in the wearable. In other examples, one or more posture sensors, air pressure sensors, gyroscopes, or magnetometer sensors or equivalent actigraphy sensors may be used to monitor the patient. Additionally, other vital signs (such as respiration rate, blood pressure, PPG waveform, body temperature) may be used to detect patient context. The received non-geospatial sensor data may comprise data relating to a predetermined timespan such as 24 hours. The non-geospatial sensor data may comprise timeseries data.

The wearable monitoring device, also called a wearable device or simply a wearable, may comprise any device such as a chest patch, wristwatch, in-ear device, or chest strap capable of monitoring the patient's vital signs.

The processing comprises preprocessing the raw non-geospatial sensor data to prepare it for the classification. The preprocessing comprises deriving patient activity level data and/or posture data from the non-geospatial sensor data. Activity level data may be based for example on an ordered list of values (e.g. 0, 1, 2, 3, 4, ..). Posture data may be based for example on a non-ordered list of classes (e.g. lie, sit, stand, upright, ...). Exertion data can also be built by combining activity level data and posture data but it will be appreciated that the activity level data or posture data in separate form is usable for classifying patient context. The preprocessing comprises extracting one or more activity behavior features and/or posture behavior features from the patient activity level data and/or posture data. The preprocessing may comprise lowpass filtering the patient activity level data and/or posture data prior to the feature extraction. Additionally or alternatively, the preprocessing may comprise one or more of the following techniques: data interpolation to cope with missing values in the timeseries; data fusion to integrate multiple data sources; data cleaning to detect and correct corrupt data; data augmentation e.g. to prevent overfitting; data normalization; data transformation.

Performing the feature extraction may comprise extracting any one or more activity behavior features or posture behavior features relevant for distinguishing between patient contexts. In one example, the activity behavior features or posture behavior features comprise features describing dispersion (variability, scatter, or spread) in the patient activity level data or posture data, respectively. In particular, the activity behavior features may comprise one or more of: standard deviation of an activity level signal in the patient activity level data; range of the activity level signal; area of the activity level signal around its mean; a time duration in which the activity level signal is below a low-activity threshold; an area of the activity level signal below a low-activity threshold plus that above a high-activity threshold; a sum of the time duration of the activity level signal above a high-level threshold and that below a low-level threshold. The posture behavior features may comprise one or more of: a mean of a posture signal in the posture data; a median of the posture signal; a standard deviation of the posture signal; a range of the posture signal; and an area of the posture signal. Furthermore, composite posture and activity behavior features may be extracted, such as e.g. the mean, median, variability of activity level data in each specific patient posture. Further high-level composite features may comprise data relating to e.g. circadian rhythm (e.g. amplitude, acrophase, period, mesophase).

In one advantageous example, in which a first predefined patient context comprises the semantic patient location "in hospital" and in which a second predefined patient context comprises the semantic patient location "at home", the activity behavior features comprise an area of the activity level above a high-activity threshold and below a low-activity threshold. In this way, patients may readily be located as being in hospital or at home based on the insightful recognition that hospital patients are rarely as inactive as well as highly active as they are at home.

Processing the non-geospatial sensor data to classify it as belonging to one of at least two predefined patient contexts may comprise using any computational algorithm or trained machine learning model to perform the classification.

In one example, processing the non-geospatial sensor data to classify it as belonging to one of at least two predefined patient contexts comprises using a comparison-based algorithm to identify similarities between an input dataset (i.e. the non-geospatial sensor data in its raw or preprocessed form, e.g. the activity level data or the activity behavior features extracted therefrom, or the posture data or the posture behavior features extracted therefrom) and at least one reference dataset representing expected (raw or preprocessed) data for a patient in a particular patient context. For example, one reference dataset may be provided for the semantic patient location "in hospital" and another for "at home". The comparison-based algorithm thus identifies correspondence between measured behavior patterns in the input dataset and expected behavior patterns in the reference dataset for one or more predefined patient contexts (e.g. "in hospital", "at home"). The comparison-based algorithm is thus able to discriminate temporal patterns in the input dataset characterizing the dataset as belonging to one of the predefined patient contexts. The comparison-based algorithm may be for example a matching algorithm such as a template matching algorithm. In one example, the template matching algorithm multiples the patient activity level data or posture data with a template of weights representing an expected patient activity level pattern or expected posture pattern for a particular patient context. In an alternative, the template matching algorithm may cross-correlate the input dataset with the reference dataset. The reference dataset(s) may be obtained for example from a repository of data for a representative patient population.

Additionally or alternatively, a machine learning model may be trained to classify an input dataset (i.e. the non-geospatial sensor data in its raw or preprocessed form) as belonging to one of the predefined patient contexts. Training data may comprise the above-mentioned one or more reference datasets representing expected raw or preprocessed sensor data for a patient in respective patient contexts, obtained for example from the repository or created specifically for the purpose of training the model. In one example, a logistic regression classifier is trained to classify the extracted one or more activity or posture behavior features as belonging to one of two predefined contexts. In other examples, any other machine learning technique for processing features may be used to perform the classification, such as those involving support vector machines, random forests, Bayesian classifiers, and so on. Moreover, neural network models such as recurrent neural networks may be used to process the raw activity level data and/or posture data to output a probability of the data having been generated in a certain predefined context.

Depending on how the classifier is implemented, the step of outputting the indication of the detected patient context may comprise outputting a probability that the current patient context belongs to a certain predefined patient context, and/or a determination as to which predefined patient context the current patient context belongs. "Current patient context" here refers to the patient context captured by the non-geospatial sensor data, in other words the context in which the non-geospatial sensor data was generated.

For example, where the classifier is a probabilistic classifier, outputting the indication may comprise outputting a probability distribution over the set of available predefined contexts. Additionally or alternatively, outputting the indication may comprise executing a decision-making algorithm to select one of the predefined contexts as being the detected patient context, e.g. that associated with the highest probability or the 'best' predefined context. The indication may thus comprise the selected predefined context and/or one or more probabilities associated with respective predefined contexts.

Where the classifier is a deterministic classifier, outputting the indication may comprise outputting the predefined context determined to be 'best' predefined context as the indication.

According to a second aspect, there is provided a method of training the machine learning model of the first aspect to classify the input dataset (i.e. the non-geospatial sensor data in its raw or preprocessed form) as belonging to one of the predefined patient contexts.

According to a third aspect, there is provided a computing device comprising a processor configured to perform the method of the first or second aspect. The methods of the first and second aspects may of course be computer implemented.

According to a fourth aspect, there is provided a computer-readable medium comprising instructions which, when executed by a computing device, enable or cause the computing device to perform the method of the first or second aspect.

The invention may include one or more aspects, examples or features in isolation or combination whether or not specifically disclosed in that combination or in isolation. Any optional feature or sub-aspect of one of the above aspects applies as appropriate to any of the other aspects.

These and other aspects of the invention will be apparent from and elucidated with reference to the embodiments described hereinafter.

BRIEF DESCRIPTION OF THE DRAWINGS

A detailed description will now be given, by way of example only, with reference to the accompanying drawings, in which:

Fig. 1 illustrates a method for patient context detection;
Fig. 2A shows first exemplary activity level data;
Fig. 2B shows second exemplary activity level data;
Fig. 3A shows a receiver operating curve indicating the relative predictive value of various behavior features in discriminating patient context when using a computational classification algorithm;
Fig. 3B shows a receiver operating curve indicating the predictive value of the behavior features of Fig. 3A when using a trained machine learning model to perform the classification;
Figs. 4-7 relate to exemplary datasets illustrating differences in sensor data and behavior features derived therefrom for two different patient contexts; and
Fig. 8 illustrates a computing device that can be used in accordance with the systems and methods disclosed herein.

DETAILED DESCRIPTION

Disclosed herein are methods and systems for classifying patient context (e.g. hospital vs home) using data obtained from a wearable device. Body movement is detected using a non-geospatial sensor, such as an accelerometer or other motion sensors embedded in the wearable device. Body motion measurements are carried out continuously during the day and night to identify whether the activity behavior of the patient represents a typical hospital or home pattern. The invention is based on the insight that activity level features in the hospital are substantially different from those observed at home. In particular, the invention is based on the surprising recognition that hospitalized patients are less physically active while at the same time rarely maintaining a low level of activity compared to when they are at home. Furthermore, the circadian pattern is less pronounced in the hospital than at home. By observing the activity level over time, it is possible to discriminate those temporal patterns occurring typically in the hospital from those occurring at home.

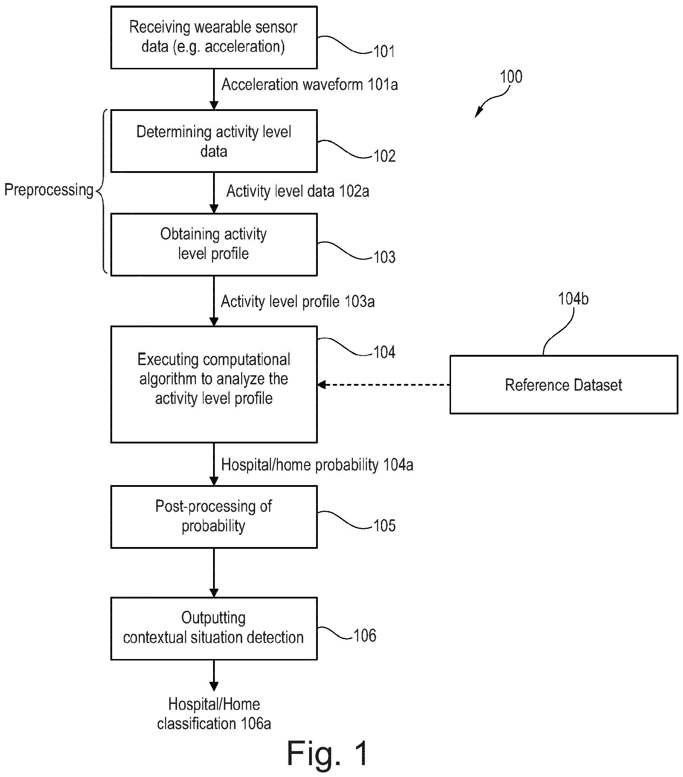

Fig. 1 illustrates a method for patient context detection according to the present disclosure. Broadly speaking, the method comprises: receiving, at step 101, non-geospatial sensor data from at least one sensor of a wearable monitoring device worn by a patient; detecting, at step 104, patient context by processing the non-geospatial sensor data to classify it as belonging to one of at least two predefined patient contexts; and outputting, at step 106, an indication of the detected patient context. In this way, the methods and systems disclosed herein advantageously provide for automatic detection of whether the patient is in hospital or at home without the need for any patient input or geolocation positioning system. These steps of the method, along with various optional features, will now be described in further detail. The method may be performed by any suitable computing device, such as that described below.

In the method described with respect to Fig. 1, the data received at step 101 comprises a waveform 101a or timeseries received from a sensor of the wearable device worn by the patient for the purpose of monitoring physical activity. Use of a wearable sensor confers unobtrusiveness and convenience for long-term use. In other examples, posture, air pressure, gyroscope, magnetometer or equivalent actigraphy sensor data may be used in addition to or instead of the acceleration data. One example of a wearable device suitable for use in conjunction with the methods and systems disclosed herein is the Healthdot device provided by Philips.

The data received at step 101 is preprocessed at steps 102 and 103 to prepare it for classification.

At step 102, the method comprises determining an activity level of the patient, for example by calculating an activity level metric from the acceleration signal waveform 101a. For example, the activity level can be expressed as the standard deviation or the interquartile range of the acceleration signal in intervals of predefined length (e.g. 5 seconds). Thus, patient activity level data 102a is derived from the received acceleration waveform 101a. The activity level data 102a, comprising a sequence of activity level metrics, may also be referred to as an activity level sequence.

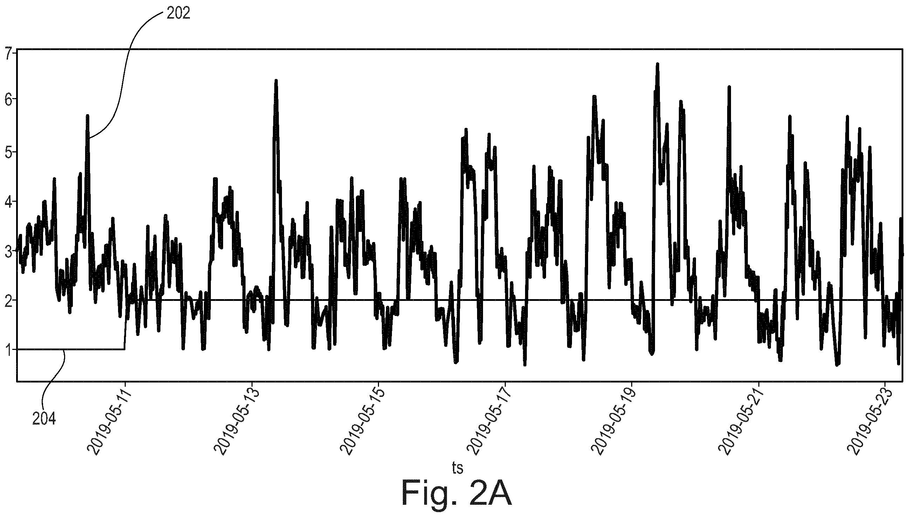

Fig. 2A shows first exemplary activity level data recorded for a patient who was hospitalized for 2 days before being discharged to home. Trace 202 shows the activity level data, while trace 204 indicates the patient context using the values 1 and 2 to represent the semantic locations "in hospital" and "at home", respectively. As can be seen, the activity level pattern observed for the patient context "at home" is very different compared to that measured for "in hospital". In particular, the activity level pattern for "at home" is characterized by its increased dispersion, including both higher highs and lower lows than that for "in hospital".

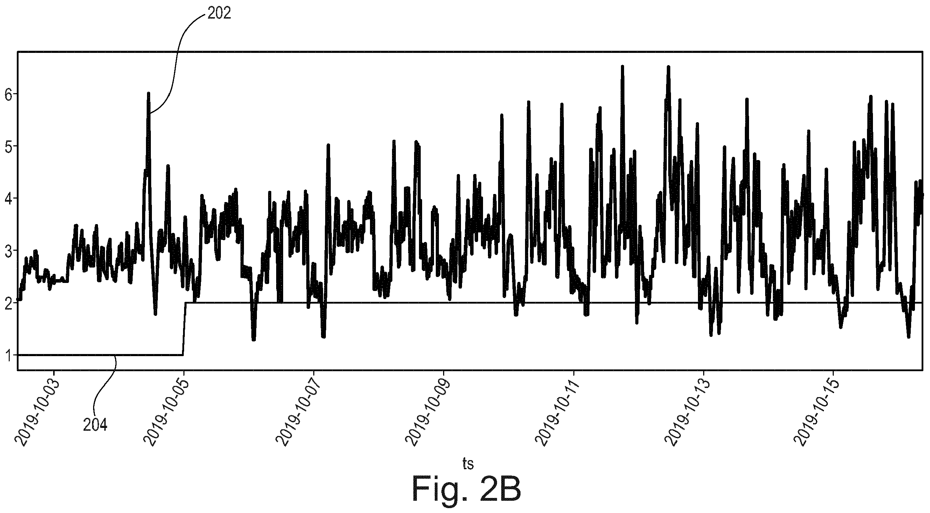

Fig. 2B shows second exemplary activity level data recorded for a patient who was hospitalized for 3 days before being discharged. The activity level pattern follows a circadian rhythm like the alternation between wake and sleep period as reflected by the activity level much more prominently in the patient context "at home" compared to "in hospital".

Referring again to Fig. 1, the method comprises obtaining, at step 103, an activity level profile 103a using the activity level data 102a. For example, feature extraction may be performed to extract one or more activity behavior features from the patient activity level data 102a. The activity level data 102a may be lowpass filtered (using e.g. a moving average filter with 30 minute windows) over a long time window (for example 24 hours), which allows trends in the daily activity level of the patient to be captured. The activity level is then processed to extract features that can characterize or discriminate the activity behavior of the patient. The extracted features together form the activity level profile 103a of the patient. These features include for example the standard deviation of the activity level over 24 hours, the range, the area of the activity level around the mean activity level, the time duration in which the activity level is minimal and below a threshold, and the area of the activity level below a low-activity threshold and above a high-activity threshold. This last feature is particularly useful as hospitalized patients are rarely completely inactive as well as highly active, which instead happens regularly at home.

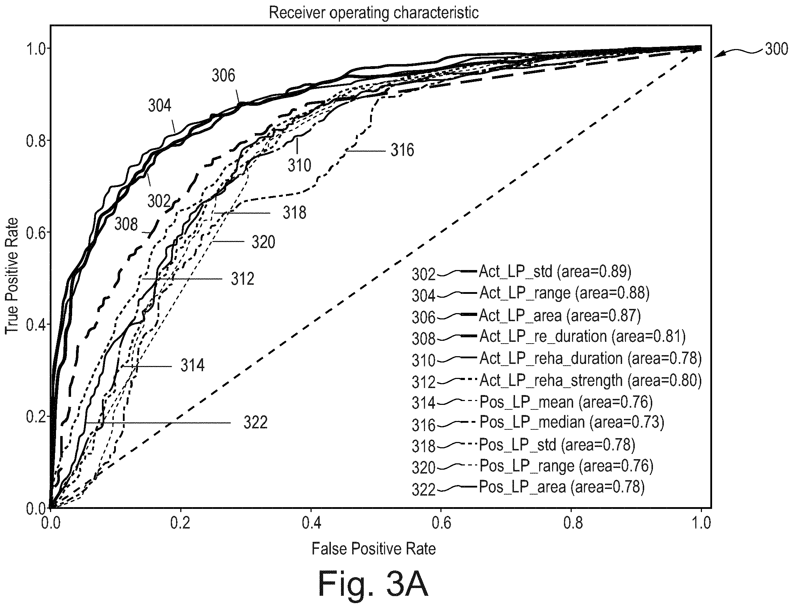

Fig. 3A shows a receiver operating curve 300 indicating the predictive value or area under the receiver-operative curve of various behavior features in discriminating the patient context "in hospital" from "at home", using a single feature classifier based on threshold rules, e.g. feature > threshold then "at home" else "in hospital". The activity behavior features shown comprise: standard deviation 302 of the activity level (Act_LP_std); range 304 of the activity level (Act_LP_range); area 306 of the activity level around its mean (Act_LP_area); time duration 308 in which the activity level signal is below a low-activity (rest) threshold (Act_LP_re_duration); time duration 310 in which the activity level signal is below the rest threshold plus time duration in which the activity level is above a high-activity threshold (Act_LP_reha_duration); area 312 of activity level below the rest threshold plus the area above the high-activity threshold (Act_LP_reha_strength). Posture behavior features 314-322 are described below. It will be noted that some or all of these behavior features comprise features describing dispersion. "LP" refers here to features derived from the lowpass filtered data.

Referring back to Fig. 1, the method comprises, at step 104, executing a computational algorithm (or computational model) to analyze the input dataset so as to classify it as belonging to the patient context "in hospital" or "at home".

In one implementation, the computational algorithm takes as input the activity behavior features as defined by the activity level profile 103a and processes them to determine whether the patient is "in hospital" or "at home". For example, the activity behavior features are processed using the computational algorithm to determine the correspondence with a reference dataset 104b for the home or for a hospital stay. Here, the reference dataset 104b comprises an expected activity level profile for "in hospital" and/or "at home", for example in the form of one or more thresholds pertaining to respective activity behavior features. A single feature classifier may then be used to perform a binary classification of the input dataset by comparing the features to the thresholds based on threshold rules: e.g. feature > threshold (then "at home" else "in hospital"). This represents one form of comparison algorithm. Alternatively, multiple feature classifiers may be used which apply combinations of such rules.

In addition to or instead of taking the activity level profile 103a as input data, the computational algorithm may process the activity level data 102a. In another implementation, the computational algorithm may comprise a template matching algorithm configured to multiply the input activity level data 102a with a template of weights (w[i]) describing the expected activity level during a home stay (e.g. captured before hospital admission, or derived from a repository of data for a representative patient population). For example, a matching value $M_{HOME}$ (or level of agreement) between the activity level data 102a (ACL[i] constituted of Ni sample values) and an activity level template for the patient context "at home" ($W_{HOME}$ [i] of Ni sample values) may be computed according to the equation:

$$M_{HOME} = ( \Sigma i: 0 \dots Ni ( w_{HOME}[i] \times ACL[i] ) ) / Ni.$$

In this case, the reference dataset 104b comprises the activity level template for "at home" and/or "in hospital". The determination of $M_{HOME}$ in this example, and/or a corresponding determination of $M_{HOSPITAL}$, may be taken as the hospital/home probability 104a shown in Fig. 1.

In another implementation, a computational algorithm is executed to classify the input dataset as belonging to the patient context "in hospital" or "at home" by measuring correlation or cross-correlation between the measured activity level data and expected activity level data for "in hospital" and/or "at home", or by multiplying the measured activity level data by the expected activity level data. The higher the sum of the multiplication, the larger the correlation, or the larger the maximum cross-correlation, the larger the probability that the measured activity level is typical for the respective patient context.

It will be understood that the use of a computational algorithm is optional and that a machine learning method may alternatively or additionally be applied to classify an input dataset as belonging to "at home" vs "in hospital".

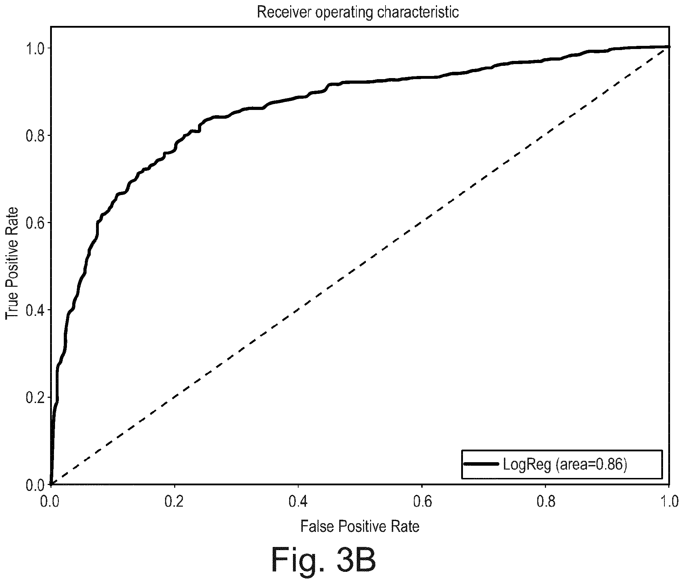

In one example, the input dataset comprises the activity behavior features of the activity level profile 103a. In this example, a logistic regression classifier may be used to perform the classification. Fig. 3B shows the classification accuracy in distinguishing between input data for "in hospital" vs "at home" when using a logistic regression classifier operating on the activity behavior features indicated in Fig. 3A. The classifier may be trained on the basis of expected activity behavior features or posture behavior features, such as those forming part of the reference dataset 104b.

In another example, the input data comprises the activity level data 102a. In this example, the activity level data 102a may be processed using a deep learning algorithm such as a recurrent neural network (long-short term memory layer, gated recurrent unit, etc.). This type of neural network layer is particularly suited to discovering the peculiar temporal patterns in the input dataset to represent an output value such as a probability of "at home" vs "in hospital". The neural network may be trained on the basis of expected activity level data or posture data for at least one of the predefined contexts.

Referring yet again to Fig. 1, postprocessing of the computed probability or probabilities is optionally performed at step 105. For example, performing postprocessing may comprise adjusting the probabilities to account for temporal trends. For example, the postprocessing may comprise a further change in probability if for example an increasing or decreasing trend in (e.g. day-to-day change) the probability is measured. The rationale is that an increasing trend is less likely to be observed compared to erratically high probabilities and may indicate that the patient is transitioning to a different context. The output of the postprocessing comprises a probability value but adjusted by some correction factor dependent on the temporal trend, such as day-by-day change, or absolute values in probability compared to certain thresholds, and so on.

Finally, a binary decision between the patient contexts "in hospital" and "at home" is made by processing the probability values, at step 106, to output the decision 106a. For example, the probability may be compared to a predefined threshold indicating confidence that the patient context is "in hospital" or "at home". The decision threshold may also be modified according to past classification output. For example, if the patient context has previously been classified as "at home", there is high likelihood that also the next decision will be "at home", so the home-decision threshold may be lowered for the following day, accordingly.

Although the above description uses the acceleration waveform 101a obtained using an acceleration sensor of the wearable device to derive the activity level data 102a and thereby the activity level profile 103a, it will be understood that the same or similar features could be extracted from posture data captured using a posture sensor, e.g. the same motion or acceleration sensor used to capture the acceleration waveform 101a. For example, Fig. 3A shows posture behavior features comprising: the mean 314 of a posture signal in the posture data; the median 316 of the posture signal; the standard deviation 318 of the posture signal; the range 320 of the posture signal; and the area 322 of the posture signal.

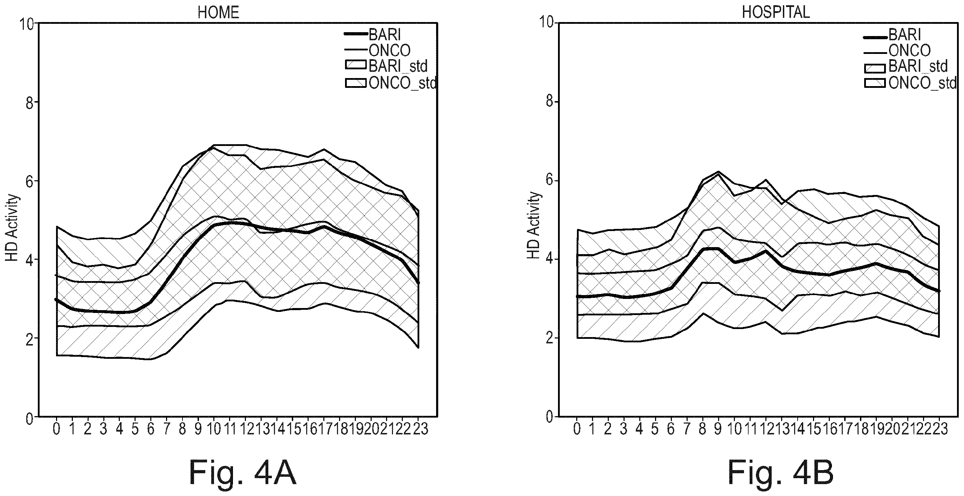

Referring now to Figs. 4-7, the following disclosure pertains to exemplary datasets illustrating the difference in patterns for the two patient contexts "at home" and "in hospital".

Figs. 4A and 4B show exemplary activity level data 102a for the patient contexts "at home" and "in hospital", respectively. "BARI" represents the average accelerometer data obtained from patients after bariatric surgery, while "ONCO" represents the average accelerometer data obtained from oncology patients, with "BARI_std" and "ONCO_std" representing activity level data in the form of the standard deviation in the respective accelerometer signal in intervals of predefined length, as described above. As can be seen, the activity pattern for "at home" for both patients shows greater dispersion over 24 hours than that for "in hospital".

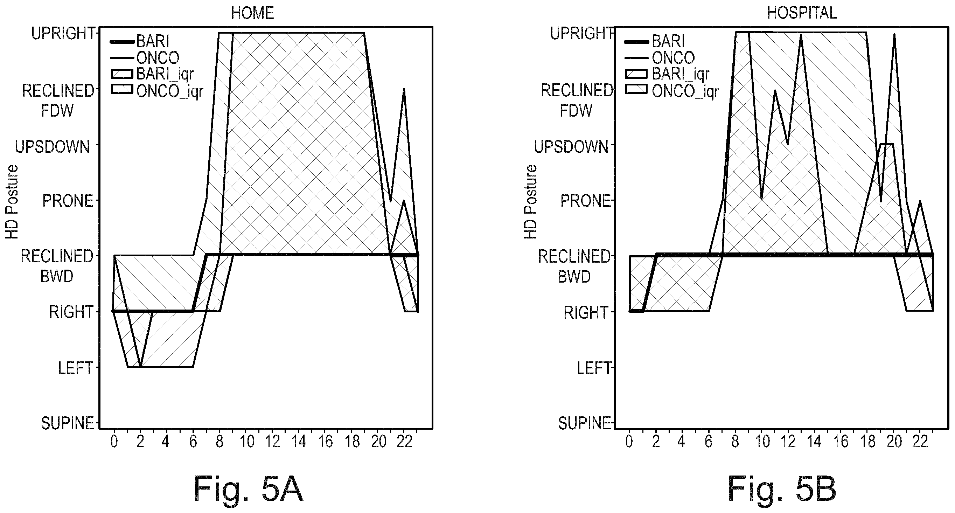

Figs. 5A and 5B show exemplary posture data for the same two patient groups. Here, "BARI_iqr" and "ONCO_iqr" represent interquartile range in the respective posture type distribution, which have been used to generate the posture data. It can be seen that both patients spend a greater proportion of the day in the posture "RECLINED BWD" when in hospital, while the interquartile range is much higher when at home.

The datasets shown in Figs. 4A, 4B, 5A, and 5B may be used as expected activity level data or expected posture data, e.g. forming part of the reference dataset 104b, for performing the computations and/or for training machine learning models, as described above.

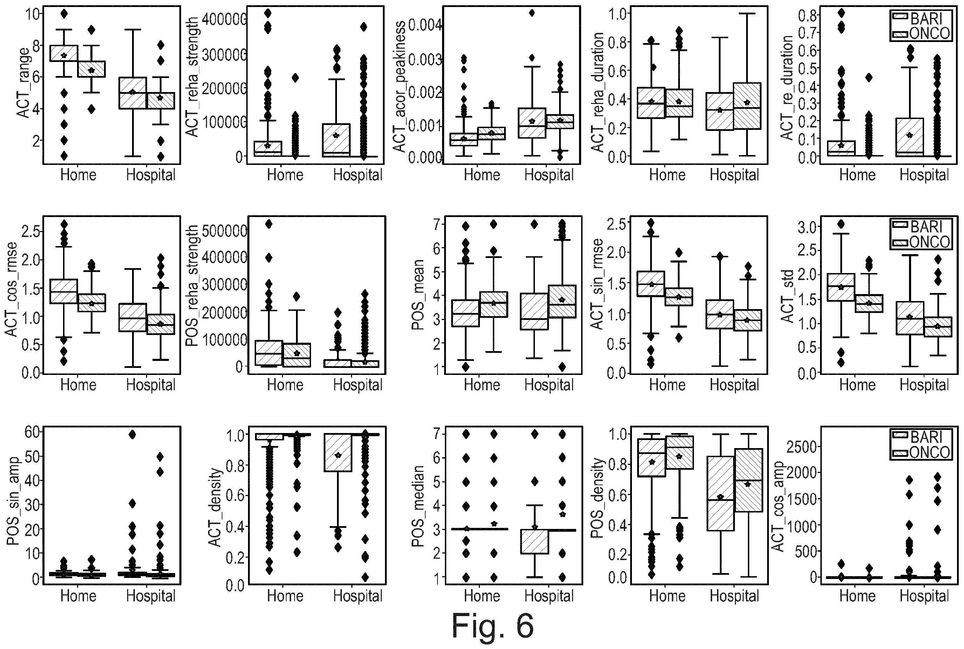

Fig. 6 illustrates numerous examples of expected activity behavior features and expected posture behavior features upon which the above-described classification may be based. These features may form part of the reference dataset 104b, for example being used to derive the above-described thresholds for classification rules, and/or for training the machine learning models. In addition to those already described above, the features include: "ACT_acor_peakiness", the difference between peak amplitude and average amplitude of the autocorrelation function; "ACT_sin_rmse", the root-mean-squared error for the fitting of a sin function fitting the activity level data, along with its counterpart "ACT_cos_rmse"; and "sin_amp" and "cos_amp", the amplitude coefficient of a sin or cos fitting function applied to the activity level data or posture data. In each case, clear distinctions between the patient contexts "at home" and "in hospital" may be observed.

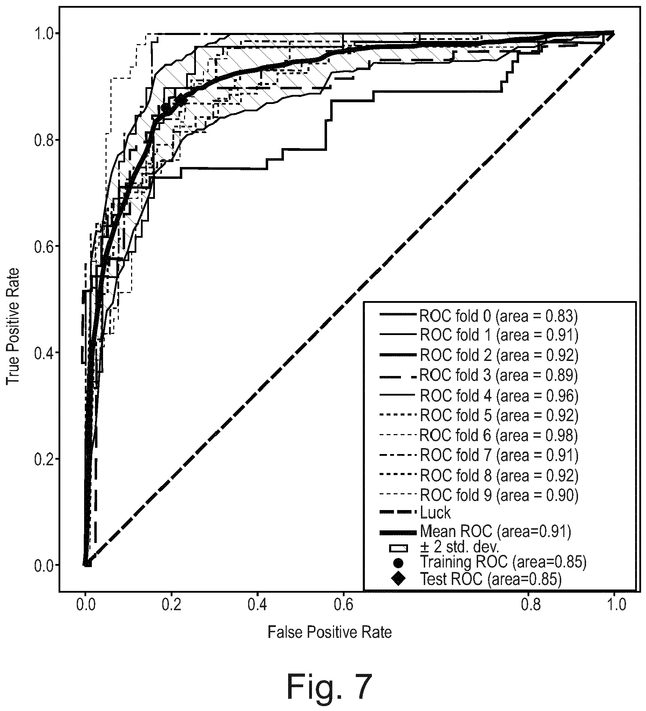

Fig 7. shows the classification accuracy of a logistic regression model using the features presented in Fig 6. In the table below, "test" and "training" indicate separate datasets used to validate and develop a logistic regression classifier (LogReg), respectively, as follows:

| Model type | LogReg | | | | |
|---|---|---|---|---|---|
| Parameter | TRAIN | CV | | | TEST |
| Parameter | *ALL* | *ALL* | | | *ALL* |
| Accuracy | 85.2 | 84.1 | +/- | 4.4 | 84.0 |
| ROC AUC | 0.853 | 0.91 | +/- | 0.04 | 0.848 |
| Sensitivity | 86.0 | 84.2 | +/- | 8.9 | 87.5 |
| Specificity | 84.7 | 83.7 | +/- | 7.0 | 82.1 |
| Positive PV | 76.6 | | | | 73.3 |
| Negative PV | 91.2 | | | | 92.1 |
| F1 Score | 81.0 | | | | 79.8 |
| Prevalence | 0.6 | | | | 0.6 |

The term CV indicates the classification results obtained on the training dataset after using a patient-wise leave-one-out cross validation approach. The various parameters mentioned in the table are classical performance indicators for binary classifiers in our case aimed at distinguishing home vs hospital context using features from the activity level or posture data.

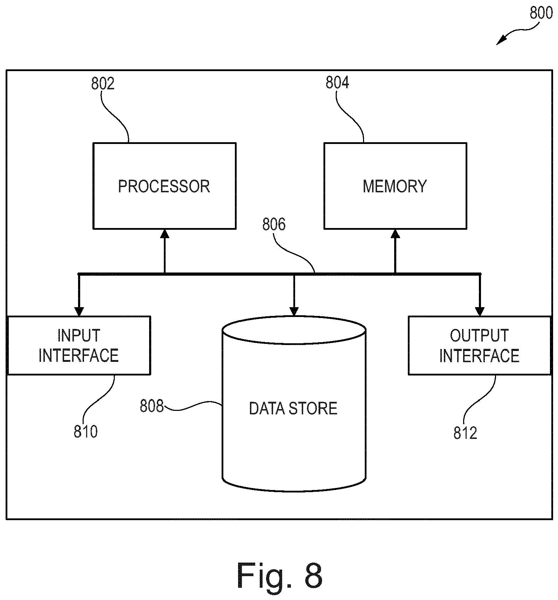

Referring now to Fig. 8, a high-level illustration of an exemplary computing device 800 that can be used in accordance with the systems and methodologies disclosed herein is illustrated. The computing device 800 includes at least one processor 802 that executes instructions that are stored in a memory 804. The instructions may be, for instance, instructions for implementing functionality described as being carried out by one or more components discussed above or instructions for implementing one or more of the methods described above. The processor 802 may access the memory 804 by way of a system bus 806. In addition to storing executable instructions, the memory 804 may also store conversational inputs, scores assigned to the conversational inputs, etc.

The computing device 800 additionally includes a data store 808 that is accessible by the processor 802 by way of the system bus 806. The data store 808 may include executable instructions, log data, etc. The computing device 800 also includes an input interface 810 that allows external devices to communicate with the computing device 800. For instance, the input interface 810 may be used to receive instructions from an external computer device, from a user, etc. The computing device 800 also includes an output interface 812 that interfaces the computing device 800 with one or more external devices. For example, the computing device 800 may display text, images, etc. by way of the output interface 812.

It is contemplated that the external devices that communicate with the computing device 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing device 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing device 800 may be a distributed system. Thus, for instance, several devices may be in communication by way of a network connection and may collectively perform tasks described as being performed by the computing device 800.

Various functions described herein can be implemented in hardware, software, or any combination thereof. If implemented in software, the functions can be stored on or transmitted over as one or more instructions or code on a computer-readable medium. Computer-readable media includes computer-readable storage media. A computer-readable storage media can be any available storage media that can be accessed by a computer. By way of example, and not limitation, such computer-readable storage media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

It will be appreciated that the aforementioned circuitry may have other functions in addition to the mentioned functions, and that these functions may be performed by the same circuit.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The word "comprising" does not exclude other elements or steps.

The indefinite article "a" or "an" does not exclude a plurality. In addition, the articles "a" and "an" as used herein should generally be construed to mean "one or more" unless specified otherwise or clear from the context to be directed to a singular form. A single processor or other unit may fulfil the functions of several items recited in the claims. Measures are recited in mutually different dependent claims can advantageously be combined. A computer program may be stored/distributed on a suitable medium, such as an optical storage medium or a solid-state medium supplied together with or as part of other hardware, but may also be distributed in other forms, such as via the internet or other wired or wireless communications systems. Any reference signs in the claims should not be construed as limiting the scope. Unless specified otherwise, or clear from the context, the phrase "A and/or B" as used herein is intended to mean all possible permutations of one or more of the listed items. That is, the phrase "X comprises A and/or B" is satisfied by any of the following instances: X comprises A; X comprises B; or X comprises both A and B.

**Claims**

1. A method for patient context detection comprising:

receiving non-geospatial sensor data from at least one sensor of a wearable monitoring device worn by a patient;
deriving patient activity level data or posture data from the non-geospatial sensor data;
extracting one or more activity behavior features from the patient activity level data, or extracting one or more posture behavior features from the posture data;
classifying the activity behavior features or posture behavior features as belonging to one of at least two predefined patient contexts; and
outputting an indication of the detected patient context.

2. The method of claim 1, wherein the preprocessing comprises lowpass filtering the patient activity level data or posture data prior to the feature extraction.

3. The method of claim 1 or 2, wherein the activity behavior features or posture behavior features comprise features describing dispersion in the patient activity level data or posture data.

4. The method of any preceding claim, wherein the activity behavior features comprise one or more of: standard deviation of an activity level signal in the patient activity level data; range of the activity level signal; area of the activity level signal around its mean; a time duration in which the activity level signal is below a low-activity threshold; an area of the activity level signal below a low-activity threshold and above a high-activity threshold; a sum of the activity level signal above a high-level threshold or below a low-level threshold; and wherein the posture behavior features comprise one or more of: a mean of a posture signal in the posture data; a median of the posture signal; a standard deviation of the posture signal; a range of the posture signal; and an area of the posture signal.

5. The method of any preceding claim, wherein a first said predefined patient context comprises the semantic patient location "in hospital" and a second said predefined patient context comprises the semantic patient location "at home".

6. The method of any preceding claim, wherein processing the non-geospatial sensor data to classify it as belonging to one of the at least two predefined patient contexts comprises using a comparison-based algorithm to identify similarities between the non-geospatial sensor data and at least one reference dataset representing expected data for a respective said predefined patient context.

7. The method of any preceding claim, wherein processing the non-geospatial sensor data to classify it as belonging to one of the at least two predefined patient contexts comprises using a template matching algorithm configured to multiply the patient activity level data or posture data with a template of weights representing an expected patient activity level pattern or expected posture pattern for the respective predefined patient context.

8. The method of any preceding claim, wherein processing the non-geospatial sensor data to classify it as belonging to one of the at least two predefined patient contexts comprises using a trained machine learning model to perform the classification.

9. The method of claim 8, wherein the machine learning model comprises a logistic regression classifier trained to classify the extracted one or more activity behavior features and/or posture behavior features as belonging to one of two said predefined contexts.

10. The method of any preceding claim, wherein outputting the indication of the detected patient context comprises outputting a probability that a current patient context belongs to a said predefined patient context, and/or a determination as to which of the predefined patient contexts the current patient context belongs.

11. A method of training a machine learning model, the method comprising training the machine learning model to classify optionally preprocessed, non-geospatial sensor data received from at least one sensor of a wearable monitoring device worn by a patient as belonging to one of at least two predefined patient contexts.

12. A computing device comprising a processor configured to perform the method of any preceding claim.

13. A computer-readable medium comprising instructions which, when executed by a computing device, enable the computing device to perform the method of any of claims 1-11.

Fig. 1

Fig. 2A

Fig. 2B

Receiver operating characteristic

302 — Act_LP_std (area=0.89)
304 — Act_LP_range (area=0.88)
306 — Act_LP_area (area=0.87)
308 — Act_LP_re_duration (area=0.81)
310 — Act_LP_reha_duration (area=0.78)
312 — Act_LP_reha_strength (area=0.80)
314 — Pos_LP_mean (area=0.76)
316 — Pos_LP_median (area=0.73)
318 — Pos_LP_std (area=0.78)
320 — Pos_LP_range (area=0.76)
322 — Pos_LP_area (area=0.78)

Fig. 3A

Fig. 3B

Fig. 4A

Fig. 4B

EP 4 109 467 A1

Fig. 5B

Fig. 5A

Fig. 6

Fig. 7

800

802

PROCESSOR

804

MEMORY

806

INPUT
INTERFACE

810

808

DATA STORE

OUTPUT
INTERFACE

812

Fig. 8

Europäisches
Patentamt

European
Patent Office

Office européen
des brevets

# EUROPEAN SEARCH REPORT

**Application Number**

EP 21 18 0580

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EHATISHAM-UL-HAQ MUHAMMAD ET AL: "C2FHAR: Coarse-to-Fine Human Activity Recognition With Behavioral Context Modeling Using Smart Inertial Sensors", IEEE ACCESS, IEEE, USA, vol. 8, 6 January 2020 (2020-01-06), pages 7731-7747, XP011765562, DOI: 10.1109/ACCESS.2020.2964237 [retrieved on 2020-01-14] * abstract * * page 7734 - page 7737 * | 1-13 | INV. G16H50/20 G16H40/67 |
| A | NWEKE HENRY FRIDAY ET AL: "Data fusion and multiple classifier systems for human activity detection and health monitoring: Review and open research directions", INFORMATION FUSION, ELSEVIER, US, vol. 46, 18 June 2018 (2018-06-18), pages 147-170, XP085510433, ISSN: 1566-2535, DOI: 10.1016/J.INFFUS.2018.06.002 * the whole document * | 1-13 | |
| A | WO 2020/243531 A2 (BIOTRILLION INC [US]) 3 December 2020 (2020-12-03) * the whole document * | 1-13 | TECHNICAL FIELDS SEARCHED (IPC) G16H |

The present search report has been drawn up for all claims

| Place of search | Date of completion of the search | Examiner |
|---|---|---|
| Munich | 6 December 2021 | Hilbig, Matthias |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 21 18 0580

This annex lists the patent family members relating to the patent documents cited in  the above-mentioned European search report.
The members are as contained in the European Patent Office EDP file on
The European Patent Office is in no way liable for these particulars which are merely given for the purpose of information.

06-12-2021

| Patent document cited in search report | Publication date | Patent family member(s) | Publication date |
|---|---|---|---|
| WO 2020243531 A2 | 03-12-2020 | NONE | |

For more details about this annex : see Official Journal of the European Patent Office, No. 12/82